# FASCICULE DE BREVET EUROPEEN

(11) **EP 2 601 911 B1**
(45) Date de publication et mention de la délivrance du brevet: **08.07.2015**
(21) Numéro de dépôt: 12195916.7
(22) Date de dépôt: 06.12.2012
(51) Int. Cl.: A61F 2/34, A61F 2/36, A61F 2/30

(54) **Composant d'une prothèse articulaire**
Komponente einer Gelenkprothese
Component of a joint prosthesis

(30) Priorité: 07.12.2011 FR 1161289
(43) Date de publication de la demande: 12.06.2013
(73) Titulaire: TORNIER, 38330 Montbonnot-Saint-Martin (FR)
(72) Inventeur: Renault, Eric, 38660 LUMBIN (FR)
(74) Mandataire: Grand, Guillaume

(56) Documents cités:
- US-A- 4 318 191
- US-A1- 2009 093 887
- US-A1- 2009 222 104
- US-A1- 2009 259 318
- US-A1- 2009 326 669

## Description

La présente invention concerne un composant d'une prothèse articulaire, cette prothèse pouvant être totale ou de resurfaçage.

L'invention s'intéresse à atténuer des bruits de grincement ou de crissement qu'émettent en service certaines prothèses articulaires. Cette émission de bruit est parfois désigner sous l'expression de « phénomène de squeaking ». Actuellement, on estime que jusqu'à 20% des patients chez lesquels a été implantée une prothèse totale de hanche se plaignent de ce phénomène qui s'avère particulièrement incommodant dans la vie de tous les jours : lorsque ces patients sollicitent leur articulation de hanche prothésée, des bruits de grincement ou de crissement s'entendent. Ces bruits résultent de vibrations d'au moins un des composants durs de la prothèse, c'est-à-dire un des composants métalliques ou céramiques, ces vibrations étant produites lorsque ce corps dur s'articule, directement ou indirectement, contre un autre composant dur de la prothèse, ou éventuellement directement contre un os du patient dans le cas d'une hémi-prothèse : en effet, pour des raisons diverses et pas nécessairement identifiées, le frottement d'une partie dure de la prothèse au niveau de l'interface d'articulation de la prothèse génère une énergie vibratoire susceptible d'exciter en vibration le corps dur d'un des composants de la prothèse, jusqu'à lui faire atteindre une fréquence de résonnance appartenant au domaine des fréquences audibles. Ce phénomène vibratoire touche notamment, dans les prothèses de hanche, aussi bien les tiges solidarisées au fémur du patient, que les cupules solidarisées à l'os du bassin.

Pour traiter ce phénomène, diverses approches ont été jusqu'à maintenant envisagées.

Ainsi, US-A-2009/0326669, sur lequel est basé le préambule de la revendication 1 annexée, propose de placer des éléments d'amortissement des vibrations dans les interfaces d'assemblage entre les composants durs d'une prothèse de hanche, c'est-à-dire, à la fois, dans l'interface d'assemblage entre une tige, solidarisée au fémur, et sa tête articulaire rapportée, et dans l'interface d'assemblage entre une cupule, solidarisée à l'os du bassin, et son insert articulaire rapporté. Ces éléments d'amortissement sont réalisés d'une seule pièce, constituée soit d'un matériau plastique, soit d'un matériau poreux de nature plastique ou métallique. Bien que séduisante sur le papier, cette solution s'avère en réalité inefficace car, eu égard aux contraintes mécaniques transmises en service entre les composants durs de la prothèse, ces éléments d'amortissement se retrouvent totalement écrasés, sans produire d'effets d'amortissement significatifs vis-à-vis des vibrations décrites plus haut.

Une autre approche, envisagée dans US-A-2009/0093887, US-A-2009/0222104 et US-A-2009/0259318, consiste à modifier la réponse vibratoire des composants durs de la prothèse, pour atténuer l'intensité des vibrations ayant une fréquence audible. Pour ce faire, la structure même du corps prothétique dont on cherche à atténuer les vibrations audibles est modifiée, notamment en y intégrant une masse d'un matériau différent du matériau dur constituant le corps prothétique, ou bien en ajourant ce corps prothétique, ou bien encore en rendant asymétriques certains profils géométriques du corps prothétique. Dans tous les cas, ces solutions s'avèrent difficiles à mettre en oeuvre car elles induisent des modifications majeures dans la conception de la prothèse. De surcroît, les performances d'atténuation ne sont pas clairement établies.

Le but de la présente invention est de proposer un composant de prothèse articulaire, dont les moyens d'atténuation des vibrations sont efficaces et simples à mettre en oeuvre.

A cet effet, l'invention a pour objet un composant d'une prothèse articulaire, tel que défini à la revendication 1.

Une des idées à la base de l'invention est de chercher à absorber les vibrations du corps dur du composant, en les transférant de manière irréversible vers une masse de dissipation de l'énergie vibratoire. Selon l'invention, ce transfert est obtenu en utilisant une masse de dissipation qui est sensiblement aussi « dure » que le corps du composant prothétique, c'est-à-dire une masse métallique ou céramique, sans évidemment que cette masse soit au contact direct du corps du composant prothétique, mais en prévoyant l'interposition entre eux d'un matériau viscoélastique : ce matériau viscoélastique transmet, de manière non linéairement élastique, à la masse les vibrations provenant du corps dur du composant prothétique, tout en les amortissant, ce qui empêche le retour de l'énergie vibratoire vers le corps du composant. L'ensemble masse-matériau viscoélastique peut être qualifié d'absorbeur vibratoire non linéaire, dans le sens où cet ensemble est à même de pomper l'énergie vibratoire provenant du corps du composant prothétique et de la dissiper rapidement. En pratique, la masse de dissipation appartenant au composant prothétique selon l'invention est petite comparativement au corps de ce composant et peut donc être facilement rapportée sur ce corps, sans affecter l'essentiel, voire la totalité des caractéristiques géométriques du corps. En service, les vibrations du corps du composant selon l'invention sont significativement atténuées, par transfert irréversible et dissipation par l'ensemble rapporté masse-matériau viscoélastique. Ces performances d'atténuation des bruits résultant de ces vibrations ont été mises en évidence par des tests expérimentaux. De surcroît, à titre d'avantage additionnel, on notera que, comme l'ensemble masse-matériau viscoélastique ne possède pas de fréquence propre, cet ensemble se cale automatiquement sur la fréquence propre du corps du composant prothétique. De plus, cet ensemble masse-matériau viscoélastique fonctionne aussi bien en régime transitoire qu'en régime établi, notamment en régime périodique que l'on rencontre typiquement lors de la marche du patient.

Des caractéristiques additionnelles avantageuses du composant prothétique conforme à l'invention sont spécifiées aux revendications dépendantes 2 à 10.

L'invention sera mieux comprise à la lecture de la description qui va suivre, donnée uniquement à titre d'exemple et faite en se référant aux dessins sur lesquels :
- la figure 1 est une vue en perspective éclatée d'un premier mode de réalisation d'un composant prothétique conforme à l'invention.
- la figure 2 est une coupe longitudinale du composant de la figure 1 à l'état assemblé ;
- les figures 3 et 4 sont des vues respectivement similaires aux figures 1 et 2, illustrant un second mode de réalisation d'un composant prothétique conforme à l'invention ;
- les figures 5 et 6 sont des vues respectivement similaires aux figures 1 et 2, illustrant un troisième mode de réalisation d'un composant prothétique conforme à l'invention ;
- les figures 7 et 8 sont des vues respectivement similaires aux figures 1 et 2, illustrant un quatrième mode de réalisation d'un composant prothétique suivant l'invention ; et
- les figures 9 et 10 sont des vues respectivement similaires aux figures 1 et 2, illustrant un cinquième mode de réalisation d'un composant prothétique conforme à l'invention.

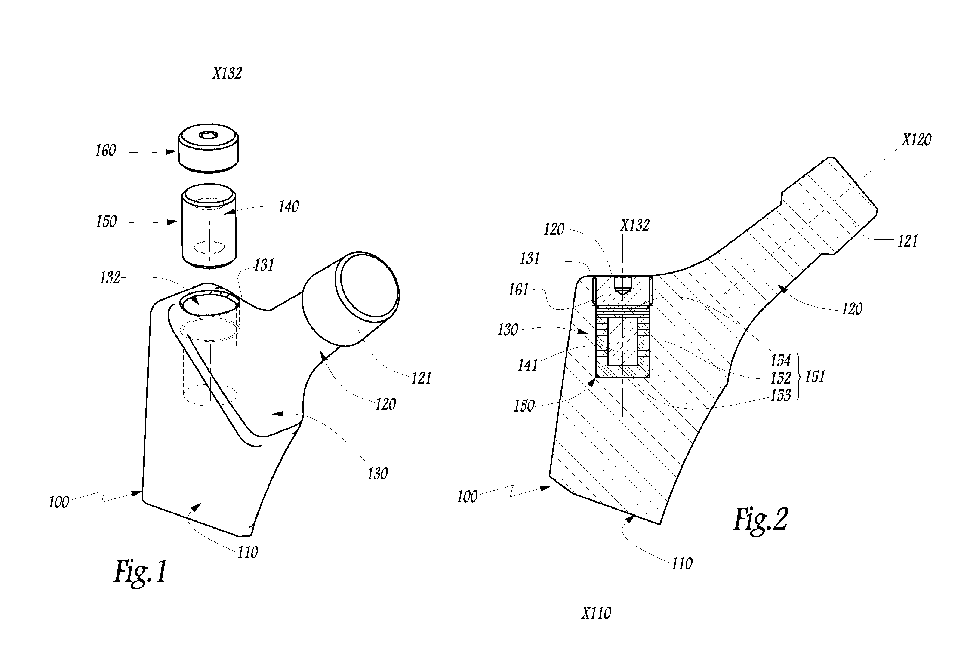

Sur les figures 1 et 2 est représentée une tige 100 d'une prothèse de hanche. De façon connue en soi, cette tige 100 comprend une partie diaphysaire 110, qui n'est représentée que partiellement sur les figures et qui, en service, est logée et retenue dans le canal médullaire d'un fémur, non représenté, d'un patient dont l'articulation de hanche correspondante est prothésée.

Par commodité, la suite de la description est orientée de sorte que les termes « supérieur » et « haut » s'entendent par rapport à une direction anatomique verticale, en étant orientée vers le haut, lorsque la tige fémorale 100 est implantée dans le fémur du patient en position debout. Ainsi, la partie diaphysaire 110 présente un axe longitudinal X110 qui, en service, s'étend, de manière globale mais non nécessairement de manière rigoureuse, à la verticale.

Comme bien visible sur les figures 1 et 2, la partie diaphysaire 110 se prolonge vers le haut par un col 120 constituant la partie terminale haute de la tige 100. Ce col 120 présente un axe longitudinal X120 qui est incliné par rapport à l'axe X110, par exemple d'environ 130°. L'angulation entre la partie diaphysaire 110 et le col 120 n'est pas limitative de la présente invention.

De manière connue en soi, l'extrémité supérieure du col 120 forme un tronc de cône 121, de type cône morse et auquel une tête prothétique hémisphérique, non représentée, est destinée à être assemblée. La tête prothétique précitée est destinée, de façon connue en soi, à s'articuler avec l'os du bassin du patient pour rétablir l'articulation de hanche, plus précisément soit directement avec le cotyle naturel de l'os du bassin, la prothèse de hanche correspondante, qui inclut la tige 100 et la tête précitée, étant alors généralement qualifiée d'hémi-prothèse de hanche, soit avec une cupule prothétique ou un ensemble de cupules prothétiques, qui remplace le cotyle de l'os du bassin, la prothèse de hanche correspondante, qui inclut la tige 100, la tête précitée et la ou les cupules prothétiques, étant alors qualifiée de prothèse totale.

La tige 100 inclut une zone 130 formant la liaison entre la partie diaphysaire 110 et le col 120. Dans l'exemple de réalisation considéré sur les figures 1 et 2, cette zone de liaison 130 est épaulée, en présentant une surface supérieure 131 sensiblement plane, qui s'étend transversalement, voire perpendiculairement à l'axe X110. La présence de la surface épaulée 131 n'est pas limitative de la présente invention.

Dans l'exemple de réalisation considéré sur les figures, la partie diaphysaire 110, le col 120 et la zone de liaison 130 forment une pièce monolithique, réalisée en un matériau dur, c'est-à-dire en métal ou en céramique. On notera que, dans le présent document, le terme « métal » désigne aussi bien une nuance métallique pure, qu'un alliage métallique. De la même façon, le terme « céramique » vise aussi bien des formulations de céramique monocomposant ou multicomposant. A titre de variante non représentée, le corps de la tige 100 peut être réalisé en plusieurs parties distinctes qui, en service, sont assemblées rigidement les unes aux autres, ces différentes parties pouvant être réalisées en des matériaux durs respectifs qui sont différents les uns des autres : à titre d'exemple, le col 120 peut être réalisé sous forme d'une pièce métallique qui est solidarisée rigidement à la zone de liaison 130, réalisée en céramique et venue de matière avec la partie diaphysaire 110. Ainsi, plus généralement, le corps de la tige 100, qui, dans l'exemple de réalisation considéré sur les figures, consiste en la pièce monolithique incluant la partie diaphysaire 110, le col 120 et la zone de liaison 130, est réalisé sous forme d'une seule pièce ou d'un assemblage rigide de plusieurs pièces, constituées de métal et/ou de céramique.

La tige 100 comprend par ailleurs une masse 140 réalisée en métal ou en céramique, autrement dit en un matériau présentant une dureté similaire à celle du ou des matériaux constituant le corps de la tige 100. Le matériau constituant la masse 140 peut être identique au matériau constituant le corps de la tige 100 ou, dans le cas d'un corps de tige incluant plusieurs matériaux, est identique à l'un de ces matériaux, de préférence au matériau présent en plus grande quantité dans le corps de tige.

En pratique, la masse 140 est significativement plus petite que la valeur de masse du corps de la tige 100. A titre d'exemple non limitatif, cette masse 140 est dix fois moindre que la valeur massique du corps de la tige 100.

Dans l'exemple de réalisation considéré sur les figures, la masse 140 se présente sous la forme d'un cylindre plein 141, à base circulaire. Ce cylindre 141 est rapporté sur le corps de la tige 100, en étant logé en totalité à l'intérieur d'une cavité 132 délimitée par la zone de liaison 130, en s'étendant en creux depuis la surface épaulée 131. Dans l'exemple de réalisation considéré ici, cette cavité 132 présente une forme globalement cylindrique, à base circulaire, le cylindre 141 de la masse 140 étant agencé de manière sensiblement co-axiale à l'intérieure de cette cavité.

Comme bien visible sur les figures 1 et 2, la masse 140 n'est pas agencée dans la cavité 132 de manière à établir un contact avec l'une des parois de cette cavité. Au contraire, un matériau viscoélastique 150 est interposé entre la masse 140 et le corps de la tige 100, en l'occurrence sa zone de liaison 130 dans le mode de réalisation considéré sur les figures. Ce matériau viscoélastique 150 se présente ici sous la forme d'un cylindre creux 151 incluant, d'un seul tenant, à la fois :
- une partie courante 152 à section transversale annulaire, dont la section intérieure est circulaire et ajustée sur le diamètre extérieur de la section transversale du cylindre 141, tandis que la section extérieure de cette partie courante 152 du cylindre 151 est circulaire et ajustée sur le diamètre intérieur de la section transversale de la cavité 132 délimitée dans la zone de liaison 130, et
- deux disques plats 153 et 154, qui ferment respectivement les deux extrémités axiales opposées de la partie courante 152.

Ainsi, suivant l'axe longitudinal central X132 de la cavité 132, qui, dans l'exemple de réalisation considéré ici, est sensiblement parallèle à l'axe X110 de la partie diaphysaire 110, le disque d'extrémité 153 du cylindre 151 du matériau viscoélastique 150 est interposé entre la masse 140 et le fond de la cavité 132, tandis que le disque d'extrémité 154 recouvre l'extrémité supérieure de la masse 140, en étant interposé entre cette masse 140 et un bouchon 160 de fermeture de la cavité, ce bouchon 160 étant décrit plus en détail ci-après. Dans le même temps, la partie courante 152 du cylindre 151 du matériau viscoélastique 150 est, radialement à l'axe X132, interposée entre la masse 140 et la paroi latérale de la cavité 132.

Autrement dit, le matériau viscoélastique 150 enrobe toute la surface extérieure de la masse 140, en comblant en totalité l'espace libre séparant, aussi bien axialement que radialement, cette masse 140 vis-à-vis des parois de la cavité 132. Aussi, eu égard à la complémentarité de formes totale entre la masse 140 et le matériau viscoélastique 150 l'enrobant, on comprend que cette masse 140 est retenue fixement par rapport au matériau viscoélastique 150. De même, eu égard à la complémentarité de formes entre le matériau viscoélastique 150 et la cavité 132, ce matériau viscoélastique est retenu à l'intérieur de cette cavité 132 : plus précisément, il est retenu fixement, d'une part, vers le bas par le fond de cette cavité et, d'autre part, transversalement par la paroi latérale de cette cavité. Le matériau viscoélastique 150 est en outre retenu vers le haut, par rapport à la cavité 132, par le bouchon 160 : dans l'exemple de réalisation considéré ici, le bouchon 160 consiste essentiellement en un disque 161 qui est adapté pour être rapporté et mis en prise mécanique avec le débouché vers le haut de la cavité 132. A titre d'exemple, comme représenté sur les figures, le disque 161 que forme le bouchon 160 est à même d'être vissé directement dans le débouché supérieur de la cavité 132, qui est taraudé à cet effet. On comprend que la mise en place, en l'occurrence le vissage, du bouchon 160 permet d'immobiliser vers le haut le matériau viscoélastique 150 vis-à-vis de la cavité 132.

Avantageusement, pour des raisons spécifiées plus loin, les aménagements permettant de mettre en prise mécaniquement le bouchon 160 sur le corps de la tige 100, en l'occurrence sur sa zone de liaison 130, sont conçus pour, en plus d'immobiliser le matériau viscoélastique 150, autoriser la mise en compression d'au moins une partie de ce matériau viscoélastique 150 : dans l'exemple de réalisation considéré sur les figures, on comprend que, moyennant un dimensionnement ad hoc de la coopération entre le filetage du bouchon 160 et le taraudage du débouché de la cavité 132, le bouchon 160 est à même de comprimer, suivant l'axe X132, les disque d'extrémité 153 et 154 du cylindre 151 du matériau viscoélastique 150, par écrasement relatif contre le corps de la tige 100 et contre la masse 140.

Lorsque la tige 100 est implantée et en service, c'est-à-dire lorsque la tête prothétique, assemblée au tronc de cône 121 de son col 120, coopère en s'articulant avec le cotyle, éventuellement prothésé, de l'os du bassin du patient, les frictions générées à l'interface de contact entre la tête prothétique et le cotyle précité induisent une énergie vibratoire, en particulier dans le cas où la tête prothétique est réalisée en un matériau dur et/ou le cotyle est prothésé par un ou plusieurs composants durs. Cette énergie vibratoire se transmet à la tige 100 et fait courir le risque d'exciter en vibration son corps dur jusqu'à une fréquence en résonnance appartenant au domaine des fréquences audibles. En pratique, cette mise en vibration est d'autant plus fréquente et significative en raison de la forme allongée de la tige 100.

Grâce à la masse 140 et au matériau viscoélastique 150, les vibrations précitées sont grandement, voire totalement atténuées. En effet, le matériau viscoélastique 150 transmet ces vibrations à la masse 140 de manière non linéairement élastique, c'est-à-dire à la façon d'un ressort non linéaire, tout en amortissant ces vibrations. Ce transfert de vibrations du corps de la tige 100 à la masse 140 par le matériau viscoélastique 150 est alors réalisé essentiellement, voire totalement de manière irréversible, c'est-à-dire sans presque, voire aucun retour possible de vibrations vers le corps de la tige 100. Autrement dit, l'énergie vibratoire provenant à la zone de liaison 130 de la tige 100 est pompée par le matériau viscoélastique 150 lors de sa transmission à la masse 140, en étant rapidement dissipée par interaction entre cette masse 140 et le matériau viscoélastique 150.

En pratique, on obtient des performances d'atténuation de vibration significatives en choisissant comme matériau viscoélastique 150 du caoutchouc naturel, du silicone, etc.

Avantageusement, à des fins de réglage, le matériau viscoélastique 150 est mis en compression par le bouchon 160, comme expliqué plus haut : dans ce cas, le bouchon 160 modifie les caractéristiques de raideur non linéaire du matériau viscoélastique 150.

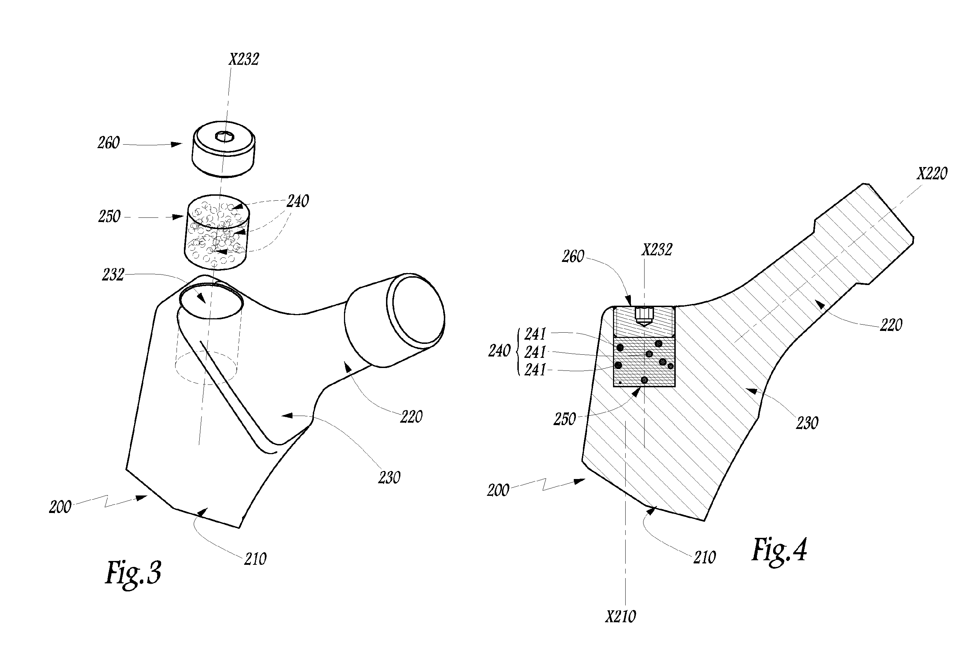

Sur les figures 3 et 4 est représenté un mode de réalisation alternatif à la tige 100, sous forme d'une tige référencée 200. Le corps dur de cette tige 200 inclut une partie diaphysaire 210 associée à un axe longitudinal X210, un col 220 associé à un axe longitudinal X220, et une zone de liaison épaulée 230 dans laquelle est délimitée une cavité en creux 232, qui sont respectivement similaires à la partie diaphysaire 110, au col 120 et la zone de liaison 130 de la tige 100.

La tige 200 se distingue de la tige 100 par la structure de sa masse 240 qui, fonctionnellement, est similaire à la masse 140 de la tige 100, mais qui, structurellement, ne se présente pas sous la forme d'un cylindre, comme le cylindre 141, mais sous la forme d'une pluralité de billes ou, plus généralement, d'éléments individuels 241, qui sont dispersés, en y étant totalement noyés, dans un matériau viscoélastique 250 fonctionnellement similaire au matériau viscoélastique 150 de la tige 100.

Le matériau viscoélastique 250 est retenu fixement dans la cavité 232, suivant des considérations similaires à la coopération entre le matériau viscoélastique 150 et la cavité 132 de la tige 100, notamment avec la présence rapportée d'un bouchon 260, qui est structurellement et fonctionnellement similaire au bouchon 160 de la tige 100.

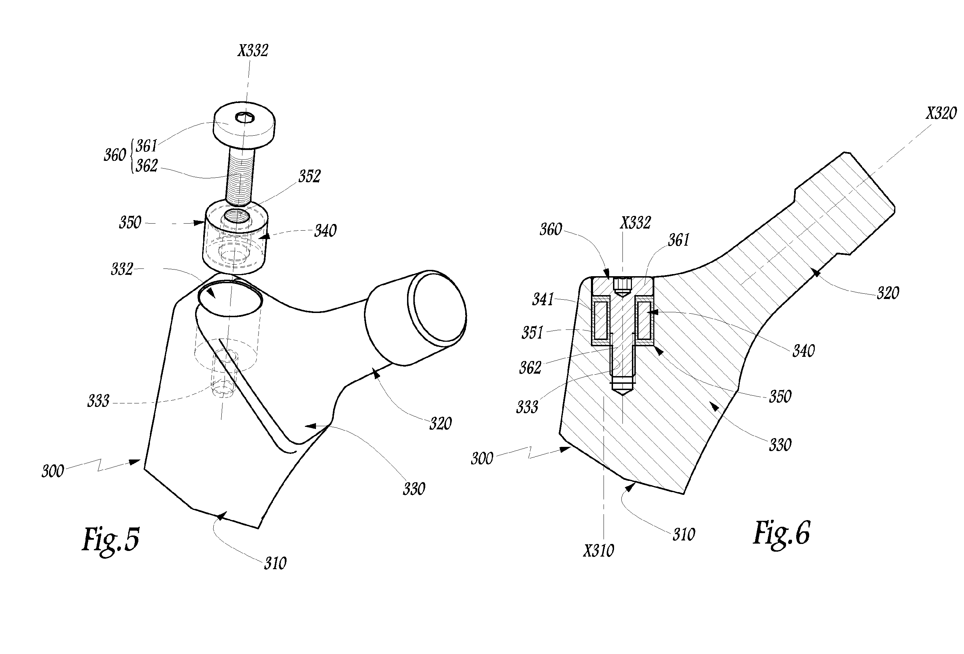

Sur les figures 5 et 6 est représenté un mode de réalisation alternatif à la tige 100, sous forme d'une tige référencée 300. Le corps de cette tige 300 comporte une partie diaphysaire 310 associée à un axe longitudinal X310, un col 320 associé à un axe longitudinal X320, et une zone de liaison épaulée 330 dans laquelle est délimitée une cavité en creux 332, qui sont respectivement similaires à la partie diaphysaire 110, au col 120 et à la zone de liaison 130 de la tige 100.

La tige 300 se distingue de la tige 100 par les formes géométriques de sa masse 340 et de son matériau viscoélastique 350, qui sont pourtant fonctionnellement similaires à la masse 140 et au matériau viscoélastique 150 de la tige 100. Plus précisément, plutôt que de présenter des formes cylindriques comme la masse 140 et le matériau viscoélastique 150, la masse 340 et le matériau viscoélastique 350 présentent des formes respectives annulaires, qui sont disposées co-axialement l'une à l'autre, l'anneau 341, à section pleine, de la masse 340 étant enrobé par l'anneau 351, à section creuse, du matériau viscoélastique 350. L'anneau 351 est agencé de manière sensiblement co-axiale dans la cavité 332, en délimitant ainsi un passage libre 352, qui est centré sur l'axe longitudinal central X332 de la cavité 332 et qui relie ainsi l'un à l'autre le fond de cette cavité et le débouché vers le haut de cette cavité.

A l'état assemblé de la tige 300, le passage précité 352 est occupé en totalité par une tige complémentaire 362 appartenant à une vis 360 de fixation du matériau viscoélastique 350 vis-à-vis de la zone de liaison 330 de la tige 300. Comme bien visible sur les figures 5 et 6, cette vis 360 comporte, en plus de la tige 362, une tête terminale 361, conformée pour fermer, par complémentarité de formes, le débouché vers le haut de la cavité 332, de sorte que la tige 362 s'étend à l'intérieur et le long du passage 352, jusqu'à être mise en prise avec un trou borgne 333 délimité en creux dans le fond de la cavité 332. A titre d'exemple, l'extrémité libre de la tige 362 coopère par vissage avec le trou borgne 333.

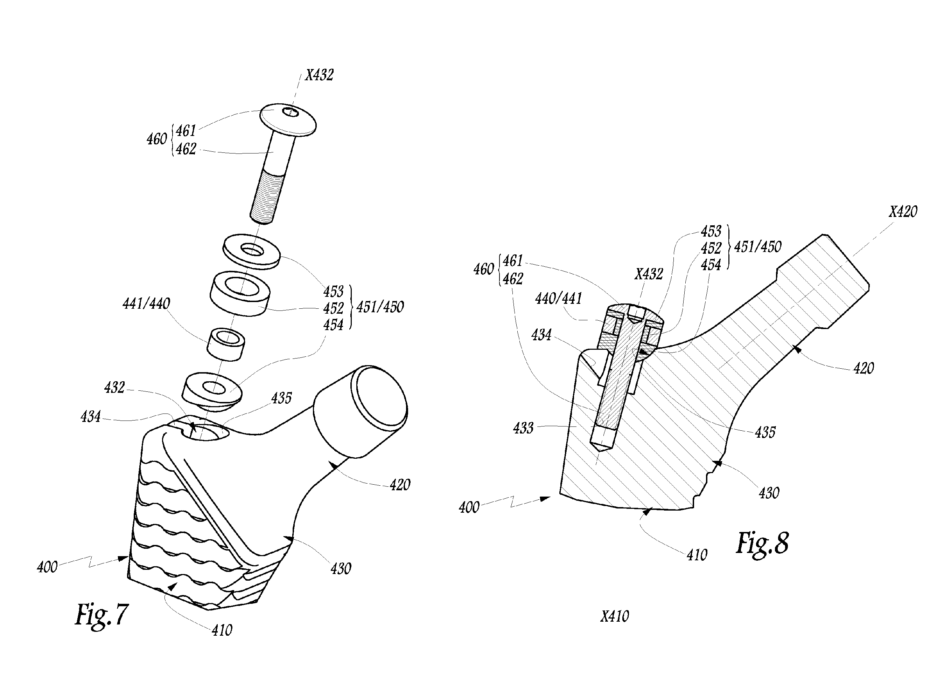

Sur les figures 7 et 8 est représenté un mode de réalisation alternatif de la tige 300, sous la forme d'une tige référencée 400. Le corps de cette tige inclut une partie diaphysaire 410 associée à un axe longitudinale X410, un col 420 associé à un axe longitudinal X420, et une zone de liaison épaulée 430 dans laquelle est délimitée une cavité en creux 432, qui sont respectivement similaires à la partie diaphysaire 310, au col 320 et à la zone de liaison 330 de la tige 300.

La tige 400 se distingue de la tige 300, à la fois, par le positionnement de sa masse 440 et de son matériau viscoélastique 450 vis-à-vis du corps de la tige 400, et par la structure de son matériau viscoélastique 450, étant pourtant remarqué que cette masse 440 et ce matériau viscoélastique 450 sont fonctionnellement similaires à la masse 340 et au matériau viscoélastique 350 de la tige 300.

Plus précisément, comme bien visible sur les figures 7 et 8, la masse 440 et le matériau viscoélastique 450 présentent des formes respectives annulaires, qui peuvent être rapprochées des formes annulaires de la masse 340 et du matériau viscoélastique 350. Cependant, comme bien visible sur la figure 8, à l'état assemblé de la tige 400, la masse 440 et l'essentiel du matériau viscoélastique 450 sont agencés à l'extérieur de la cavité 432. Plus précisément, le corps 451 constituant le matériau viscoélastique 450 inclut, sous forme de pièces élémentaires distinctes, à la fois :
- un anneau 452, qui, intérieurement, reçoit la tige 462 d'une vis 460 similaire à la vis 360, et qui, extérieurement, est ceinturé de manière ajustée par un anneau 441 constituant la masse 440,
- une rondelle d'extrémité supérieure 453, qui, intérieurement, reçoit de manière ajustée la tige 462 de la vis 460, et qui, par sa partie périphérique extérieure, s'interpose, suivant la direction de l'axe X432 de la cavité 432, entre le chant d'extrémité supérieure de l'anneau 441 de la masse 440 et la tête 461 de la vis 460, et
- une rondelle d'extrémité inférieure 454, qui, intérieurement, reçoit de manière ajustée la tige 462 de la vis 460, et qui, par sa partie périphérique extérieure, s'interpose, suivant la direction de l'axe X432, entre le chant d'extrémité inférieure de l'anneau 441 de la masse 440 et la surface supérieure épaulée 431 de la zone de liaison 430.

Sous l'action de la vis 460 lorsque la tige 462 de cette dernière est mise en prise, notamment vissée, dans un trou borgne 433 délimité en creux dans le fond de la cavité 432, l'anneau 452 et les rondelles d'extrémité 453 et 454 sont assemblés fixement les uns aux autres de manière à retenir entre eux la masse 440, tout en fixant le corps 451, ainsi assemblé, du matériau viscoélastique 450 par rapport à la zone de liaison 430, plus globalement par rapport au corps de la tige 400. Dans la mesure où la masse 440 s'étend alors à l'extérieur de la cavité 432, on comprend que le matériau viscoélastique 450 n'a pas nécessairement à recouvrir la face périphérique extérieure de l'anneau 441 de la masse 440 à des fins d'interposition vis-à-vis du corps de la tige 400. On comprend au passage la faisabilité et l'intérêt pratique d'obtenir le corps 451 du matériau viscoélastique 450 par assemblage de l'anneau 452 et des rondelles d'extrémité 453 et 454, plutôt que de prévoir d'enrober totalement la masse par le matériau viscoélastique, comme pour les tiges 100, 200 et 300.

Un autre intérêt, indépendant de celui évoqué ci-dessus, lié à l'agencement de la masse 440 et du matériau viscoélastique 450 à l'extérieur de la cavité 332 est lié à la possibilité de pouvoir, si besoin, facilement dégager ces éléments et ainsi utiliser la cavité 432 à des fins d'extraction du corps de la tige 400. En effet, la cavité 432 constitue avantageusement un logement de réception d'un ancillaire de retrait du corps de la tige 400 vis-à-vis de fémur. D'ailleurs, on comprend que cette cavité 432 peut, en réalité, être une cavité pré-existante du corps de la tige 400, c'est-à-dire une cavité connue en soi pour constituer un logement de réception d'un tel ancillaire de retrait : dans ce cas, la masse 440, le matériau viscoélastique 450 et la vis d'assemblage et de fixation 460 sont conçus pour mettre à profit la pré-existence de cette cavité 432 afin d'équiper en service le corps de la tige 400, comme sur les figures 7 et 8.

Cela explique alors pourquoi, dans le mode de réalisation considéré sur les figures 7 et 8, la cavité 432 présente des aménagements non nécessaires à la présence de la masse 440 et du matériau viscoélastique 450 : en particulier, cette cavité 432 présente, au niveau de son débouché vers le haut, une fente 434 de passage latéral pour l'ancillaire précité, ainsi qu'un évasement progressif 435 destiné, entre autres, à faciliter l'application de cet ancillaire, étant remarqué que le chant d'extrémité inférieure de la rondelle 454 du corps 451 du matériau viscoélastique 450 est conformé de manière complémentaire pour s'ajuster à cet évasement 435.

En tenant compte, entre autres, des considérations précédentes, on comprend également pourquoi l'axe X432 présente, par rapport à l'axe X41 0 de la partie diaphysaire 410, une inclinaison plus marquée que celle, qui est faible voire nulle, entre les axes X31 0 et X332 de la tige 300. Bien entendu, à titre de variante non représentée, les axes X432 et X410 pourraient ne pas être inclinés l'un par rapport à l'autre, ou être beaucoup moins inclinés l'un vis-à-vis de l'autre que sur les figures 7 et 8.

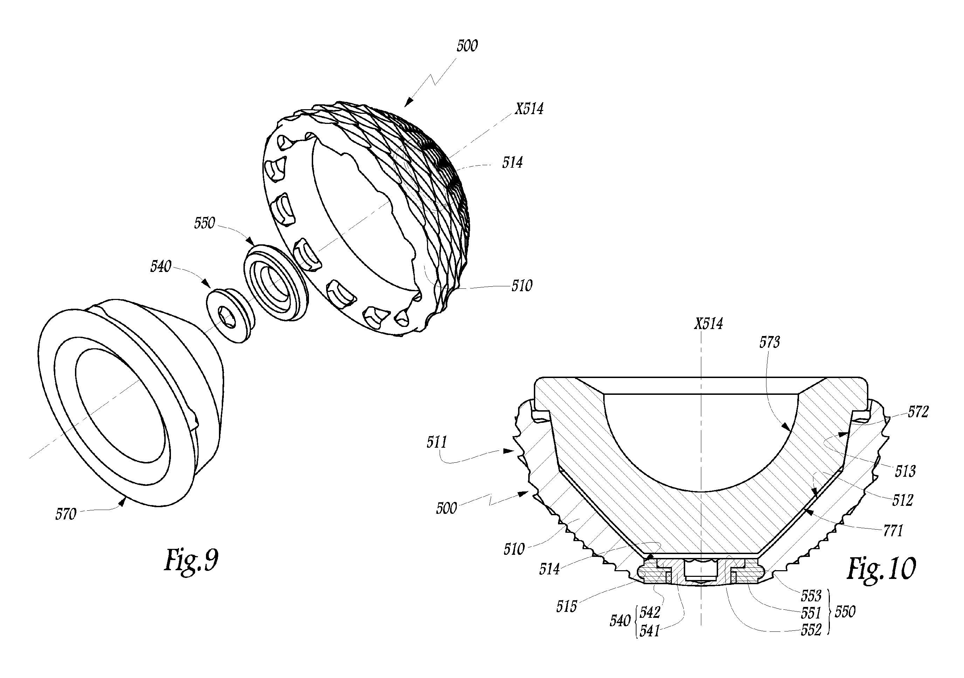

Sur les figures 9 et 10 est représentée une cupule 500 d'une prothèse de hanche, associée à un insert 570. De façon connue en soi, la cupule 500 comprend principalement un corps 510 en forme de demi-coquille globalement sphérique, destiné à être logé et retenu dans l'os du bassin du patient afin de remplacer le cotyle de cet os. Le corps 510 présente une face extérieure convexe 511 conçue pour être ancrée dans l'os du bassin, les aménagements de cette face extérieure 511 en vue de son ancrage osseux n'étant pas limitatifs de la présente invention. Le corps 510 présente également une face intérieure concave 512 adaptée pour recevoir intérieurement l'insert 570 et pour l'y immobiliser : dans l'exemple de réalisation considéré sur les figures, la face intérieure 512 inclut, dans sa partie débouchant sur l'extérieur, une partie tronconique 513, de type cône morse, et contre laquelle une surface complémentaire 572 de la face extérieure 571 de l'insert 570 est prévue pour être assemblée fixement, par coincement, comme montré à la figure 10. De manière plus générale, les aménagements de la face intérieure 512 du corps 510 aux fins d'assemblage de l'insert 570 à la cupule 500 ne sont pas limitatifs de la présente invention.

Egalement de manière connue en soi, l'insert 570 présente une face intérieure 573 creuse et sensiblement hémisphérique : en service, cette face intérieure 573 est le lieu d'articulation de la tête, naturelle ou prothétique, du fémur du patient. Ainsi, à titre d'exemple, la cupule 500 et l'insert 570 peuvent être utilisés avec l'une des tiges 100, 200, 300 et 400, sur le col de laquelle est assemblée une tête prothétique fémorale.

Le corps 510 de la cupule 500 étant réalisé en un matériau dur, c'est-à-dire en métal ou en céramique, ce corps 510 est susceptible d'être parcouru par des vibrations produites lors des mouvements d'articulation entre l'insert 570 et la tête, naturelle ou prothétique, du fémur du patient lorsque ce dernier sollicite son articulation de hanche prothésée. Ainsi, on retrouve pour la cupule 500 les considérations développées plus haut pour les tiges 100, 200, 300 et 400 : l'apparition et l'intensité de ces vibrations sont d'autant plus marquées dans le cas où l'insert 570 est, lui aussi, réalisé en un matériau dur, c'est-à-dire en métal ou en céramique. Ceci étant, des vibrations peuvent également être transmises à la cupule 500 même dans le cas où l'insert 570 est réalisé en un matériau semi-rigide, tel que du PEHD.

Quelle qu'en soit l'origine, les vibrations du corps 510 de la cupule 500 sont significativement atténuées par des moyens dédiés appartenant à la cupule 500, à savoir une masse 540 et un matériau viscoélastique 550, qui sont fonctionnellement similaires à la masse 140 et au matériau viscoélastique 150 de la tige 100. Les caractéristiques fonctionnelles de la masse 540 et du matériau viscoélastique 550 ne seront donc pas décrites plus avant, seules les caractéristiques structurelles de ces éléments faisant l'objet des explications qui vont suivre, dans la mesure où ces caractéristiques structurelles ne se retrouvent pas dans les tiges 100, 200, 300 et 400 décrites jusqu'ici.

Plus précisément, la masse 540 et le matériau élastique 550 se présente chacun sous une forme globalement annulaire, coaxiale l'une à l'autre et agencée au sommet du corps 510 de la cupule 500. A cet effet, le corps 510 délimite ainsi, dans la région de son sommet, un trou traversant 514 d'axe central X514 reliant l'une à l'autre les faces extérieure 511 et intérieure 512 de ce corps. La région centrale du trou traversant 514 est occupée par la masse 540, tandis que la partie périphérique du trou traversant est occupée par le matériau viscoélastique 550, qui s'interpose ainsi radialement entre toute la paroi latérale périphérique de la masse 540 et la paroi périphérique du trou traversant 514, comme bien visible sur la figure 10. La masse 540 et le matériau viscoélastique 550 sont retenus fixement l'un par rapport à l'autre par complémentarité de formes : dans l'exemple de réalisation considéré sur les figures, cette coopération par complémentarité de formes repose, à la fois, sur une butée axiale entre une collerette 542, en saillie de l'anneau principal 541 de la masse 540, et un épaulement complémentaire 552, délimité par l'anneau principal 551 du matériau viscoélastique 550, et sur le vissage d'au moins une partie de l'anneau 541 de la masse 540 à l'intérieur de l'anneau 551 du matériau viscoélastique 550.

Le matériau viscoélastique 550 est, quant à lui, retenu fixement à l'intérieur du trou traversant 514 du corps 510, sans nécessiter la présence de moyens de fixation additionnels, tels que sont les bouchons 160 et 260 et les vis 360 et 460 des tiges 100, 200, 300 et 400 décrites plus haut. En effet, comme bien visible sur la figure 10, l'anneau principal 551 du matériau viscoélastique 550 présente, sur sa face périphérique extérieure, un bourrelet périphérique saillant 553 adapté pour être logé et se coincer dans une rainure complémentaire 515 délimitée dans la paroi du trou 514. Bien entendu, d'autres formes de réalisation sont envisageables pour retenir fixement l'un par rapport à l'autre le corps 510 et le matériau viscoélastique 550 exclusivement par complémentarité de formes.

Divers aménagements et variantes aux tiges 100, 200, 300 et 400 et à la cupule 500 décrites jusqu'ici sont par ailleurs envisageables. A titre d'exemples :
- la zone du corps des tiges 100, 200, 300 et 400, ainsi que la zone du corps 510 de la cupule 500, dans lesquelles sont agencés les masses 140, 240, 340, 440 et 540 et les matériaux viscoélastiques 150, 250, 350, 450 et 550, ne sont pas limitées à celles envisagées dans les modes de réalisation évoqués jusqu'ici ; en effet, on comprend que cette zone d'agencement peut, en variante non représentée, être située en divers endroits du corps de la tige ou de la cupule, sans compromettre les performances d'atténuation des vibrations ; ceci étant, cette zone d'agencement est avantageusement choisie pour ne pas interférer avec des zones fonctionnelles principales, notamment préexistantes, du corps de la tige ou de la cupule ; en particulier, comme pour les tiges 100 à 400 et pour la cupule 500, cette zone d'agencement est distincte de la zone du corps des tiges ou de la cupule, par laquelle les contraintes d'articulation sont transmises à ce corps lors de la sollicitation de l'articulation prothésée du patient ; cela revient à dire que, par exemple pour la tige 100, la masse 140 et le matériau viscoélastique 150 ne sont pas agencés dans la zone du tronc de cône 121, tandis que, pour la cupule 500, cela revient à dire que la masse 540 et le matériau viscoélastique 550 ne sont pas agencés dans la zone de la partie tronconique 513 du corps 510 ;
- les enseignements techniques de l'invention sont applicables à d'autres composants prothétiques que ceux d'une prothèse de hanche ; en particulier, une masse associée à un matériau viscoélastique peut être agencée, à des fins d'atténuation de vibration, sur le corps dur d'un composant de prothèse de genou, d'un composant de prothèse d'épaule, etc. ; et/ou
- comme évoqué plus haut, l'invention trouve une application particulière au cas des prothèses totales, c'est-à-dire incluant deux composants prothétiques qui s'articulent l'un contre l'autre, en formant notamment un couple métal/métal ou un couple céramique/céramique.

## Revendications

1. Composant (100 ; 200 ; 300 ; 400 ; 500) d'une prothèse articulaire, comportant :
- un corps (110, 120, 130 ; 210, 220, 230 ; 310, 320, 330 ; 410, 420, 430 ; 510), qui est réalisé en métal ou en céramique, qui est adapté pour être solidarisé, directement ou indirectement, à un premier os d'un patient, et qui est adapté pour s'articuler, directement ou indirectement, avec un second os du patient pour rétablir une articulation avec le premier os, et
- des moyens (140, 150 ; 240, 250 ; 340, 350 ; 440, 450 ; 540, 550) d'atténuation de vibrations du corps,
**caractérisé en ce que** les moyens d'atténuation comprennent une masse métallique ou céramique (140 ; 240 ; 340 ; 440 ; 540), qui est rapportée fixement sur le corps (110, 120, 130 ; 210, 220, 230 ; 310, 320, 330 ; 410, 420, 430 ; 510), avec interposition d'un matériau viscoélastique (150 ; 250 ; 350 ; 450 ; 550).

2. Composant suivant la revendication 1, **caractérisé en ce que** la masse (140 ; 240 ; 340 ; 440 ; 540) est agencée dans une zone du corps (110, 120, 130 ; 210, 220, 230 ; 310, 320, 330 ; 410, 420, 430 ; 510), distincte de sa ou ses zones par lesquelles des contraintes d'articulation avec le second os sont transmises au reste du corps.

3. Composant suivant une des revendications 1 ou 2, **caractérisé en ce que** la masse (140 ; 240 ; 340 ; 440 ; 540) et le matériau viscoélastique (150 ; 250 ; 350 ; 450 ; 550) sont retenus fixement l'un par rapport à l'autre par complémentarité de formes.

4. Composant suivant la revendication 3, **caractérisé en ce que** le matériau viscoélastique (150 ; 250 ; 350) enrobe au moins partiellement la masse (140 ; 240 ; 340) de manière à retenir fixement l'un par rapport à l'autre la masse et le matériau viscoélastique.

5. Composant suivant l'une des revendications 3 ou 4, **caractérisé en ce que** le matériau viscoélastique (450) inclut des pièces distinctes (452, 453, 454), qui sont assemblées fixement les unes aux autres de manière à retenir entre elles la masse (440).

6. Composant suivant l'une quelconque des revendications précédentes, **caractérisé en ce que** le composant (100 ; 200 ; 300 ; 400) comporte en outre des moyens mécaniques (160 ; 260 ; 360 ; 460) pour fixer l'un à l'autre le corps (110, 120, 130 ; 210, 220, 230 ; 310, 320, 330 ; 410, 420, 430), et le matériau viscoélastique (150 ; 250 ; 350 ; 450).

7. Composant suivant la revendication 6, ; **caractérisé en ce que** les moyens mécaniques (160 ; 260 ; 360 ; 460) sont adaptés pour mettre en prise une partie correspondante du corps (110, 120, 130 ; 210, 220, 230 ; 310, 320, 330 ; 410, 420, 430), notamment par vissage, de manière à comprimer le matériau viscoélastique (150 ; 250 ; 350 ; 450) contre le corps et/ou contre la masse (140 ; 240 ; 340 ; 440).

8. Composant suivant la revendication 7, **caractérisé en ce que** ladite partie correspondante du corps (410) est un logement (432) de réception d'un ancillaire de retrait du corps vis-à-vis du premier os.

9. Composant suivant l'une quelconque des revendications 1 à 5, **caractérisé en ce que** le matériau viscoélastique (550) et le corps (510) sont conformés pour coopérer l'un avec l'autre par complémentarité de formes de manière à, à eux seuls, retenir fixement l'un par rapport à l'autre le corps et le matériau viscoélastique.

10. Composant suivant l'une quelconque des revendications précédentes, **caractérisé en ce que** le composant est une tige (100 ; 200 ; 300 ; 400) ou une cupule (500) d'une prothèse de hanche.

## Patentansprüche

1. Bauelement (100; 200; 300; 400; 500) einer Gelenkprothese, aufweisend:
- einen Körper (110, 120, 130; 210, 220, 230; 310, 320, 330; 410, 420, 430; 510), der aus Metall oder aus Keramik realisiert ist, der angepasst ist, um direkt oder indirekt mit einem ersten Knochen eines Patienten verbunden zu sein, und der angepasst ist, um direkt oder indirekt mit einem zweiten Knochen des Patienten gelenkverbunden zu sein, um ein Gelenk mit dem ersten Knochen wieder herzustellen, und
- Mittel (140, 150; 240, 250; 340, 350; 440, 450; 540, 550) zur Dämpfung von Vibrationen des Körpers,
**dadurch gekennzeichnet, dass** die Mittel zur Dämpfung eine Metall- oder Keramik-Masse (140; 240; 340; 440; 540) aufweisen, die fest an dem Körper (110, 120, 130; 210, 220, 230; 310, 320, 330; 410, 420, 430; 510) angebracht ist mit Zwischenanordnung eines viskoelastischen Materials (150; 250; 350; 450; 550).

2. Bauelement gemäß Anspruch 1, **dadurch gekennzeichnet, dass** die Masse (140; 240; 340; 440; 540) in einem Bereich des Körpers (110, 120, 130; 210, 220, 230; 310, 320, 330; 410, 420, 430; 510) angeordnet ist, der von seinem oder seinen Bereichen verschieden ist, mittels denen die Kräfte des Gelenks mit dem zweiten Knochen auf den Rest des Körpers übertragen werden.

3. Bauelement gemäß einem der Ansprüche 1 oder 2, **dadurch gekennzeichnet, dass** die Masse (140; 240; 340; 440; 540) und das viskoelastische Material (150; 250; 350; 450; 550) über Komplementarität der Formern fest aneinander gehalten sind.

4. Bauteil gemäß Anspruch 3, **dadurch gekennzeichnet, dass** das viskoelastische Material (150; 350; 350) die Masse (140; 240; 340) zumindest teilweise umgibt, um die Masse und das viskoelastische Material fest aneinander zu halten.

5. Bauteil gemäß einem der Ansprüche 3 oder 4, **dadurch gekennzeichnet, dass** das viskoelastiche Material (450) verschiedene Stücke (452, 453, 454) aufweist, die fest aneinander angebracht sind, um die Masse (440) zwischen sich halten.

6. Bauteil gemäß irgendeinem der vorhergehenden Ansprüche, **dadurch gekennzeichnet, dass** das Bauteil (100; 200; 300; 400) ferner mechanische Mittel (160; 260; 360; 460) zum aneinander Fixieren des Körpers (110, 120, 130; 210, 220, 230; 310, 320, 330; 410, 420, 430) und des viskoelastischen Materials (150; 250; 350; 450) aufweist.

7. Bauteil gemäß Anspruch 6, **dadurch gekennzeichnet, dass** die mechanischen Mittel (160; 260; 360; 460) angepasst sind, um mit einem korrespondierenden Teil des Körpers (110, 120, 130; 210, 220, 230; 310, 320, 330; 410, 420, 430) in Eingriff zu stehen, insbesondere via Gewindeeingriff, um das viskoelastische Material (150; 250; 350; 450) gegen den Körper und/oder gegen die Masse (140; 240; 340; 440) zu komprimieren.

8. Bauteil gemäß Anspruch 7, **dadurch gekennzeichnet, dass** der korrespondiere Teil des Körpers (410) eine Unterbringung (432) zur Aufnahme eines Dehnungshilfsmittels des Körpers gegenüber dem ersten Knochen ist.

9. Bauteil gemäß irgendeinem der Ansprüche 1 bis 5, **dadurch gekennzeichnet, dass** das viskoelastische Material (550) und der Körper (510) in Übereinstimmung gebracht sind, um miteinander zusammenwirken, durch Komplementarität der Formen, um, jeweilig, den Körper und das viskoelastische Materail fest aneinander zu halten.

10. Bauteil gemäß irgendeinem der vorhergehenden Ansprüche, **dadurch gekennzeichnet, dass** das Bauteil ein Schaft (100; 200; 300; 400) oder eine Pfanne (500) einer Hüftprothese ist.

## Claims

1. Component (100; 200; 300; 400; 500) of a joint prosthesis comprising:
- a body (110, 120, 130; 210, 220, 230; 310, 320, 330; 410, 420, 430; 510), made of metal or ceramic, which is suitable for being interlocked, directly or indirectly, with a first bone of a patient, and which is suitable for articulating, directly or indirectly, with a second bone of the patient to re-establish a joint with the first bone, and
- means (140, 150; 240, 250; 340, 350; 440, 450; 540, 550) of reducing vibrations of the body, **characterised in that** the means of reduction comprise a metal or ceramic mass (140; 240; 340; 440; 540) which is attached to the body (110, 120, 130; 210, 220, 230; 310, 320, 330; 410, 420, 430; 510) in a fixed manner, with a viscoelastic material (150; 250; 350; 450; 550) interposed.

2. Component according to claim 1, **characterised in that** the mass (140; 240; 340; 440; 540) is arranged in an area of the body (110, 120, 130; 210, 220, 230; 310, 320, 330; 410, 420, 430; 510) distinct from its area or areas through which joint stresses with the second bone are transmitted to the rest of the body.

3. Component according to one of claims 1 or 2, **characterised in that** the mass (140; 240; 340; 440; 540) and the viscoelastic material (150; 250; 350; 450; 550) are held in a fixed manner in relation to one another in a form-fitting manner.

4. Component according to claim 3, **characterised in that** the viscoelastic material (150; 250; 350) at least partially encases the mass (140; 240; 340) in such a way as to hold the mass and the viscoelastic material in a fixed manner in relation to one another.

5. Component according to one of claims 3 or 4, **characterised in that** the viscoelastic material (450) includes distinct parts (452, 453, 454) which are assembled in a fixed manner with one another in such a way as to hold the mass (440) between them.

6. Component according to any of the preceding claims, **characterised in that** the component (100; 200; 300; 400) additionally comprises mechanical means (160; 260; 360; 460) to fix the body (110, 120, 130; 210, 220, 230; 310, 320, 330; 410, 420, 430) and the viscoelastic material (150; 250; 350; 450) against one another.

7. Component according to claim 6, **characterised in that** the mechanical means (160; 260; 360; 460) are suitable for engaging a corresponding part of the body (110, 120, 130; 210, 220, 230; 310, 320, 330; 410, 420, 430), particularly by screwing, in such a way as to compress the viscoelastic material (150; 250; 350; 450) against the body and/or against the mass (140; 240; 340; 440).

8. Component according to claim 7, **characterised in that** the said corresponding part of the body (410) is a casing (432) for receiving a withdrawal ancillary means of the body with regard to the first bone.

9. Component according to any of claims 1 to 5, **characterised in that** the viscoelastic material (550) and the body (510) are configured to cooperate with one another in a form-fitting manner such that they alone hold the body and the viscoelastic material in a fixed manner in relation to one another.

10. Component according to any of the preceding claims, **characterised in that** the component is a rod (100; 200; 300; 400) or a cup (500) of a hip prosthesis.
